# EUROPÄISCHE PATENTSCHRIFT

(11) **EP 2 598 436 B1**
(45) Veröffentlichungstag und Bekanntmachung des Hinweises auf die Patenterteilung: **24.09.2014**
(21) Anmeldenummer: 11740562.1
(22) Anmeldetag: 15.07.2011
(51) Int. Cl.: A61K 8/22, A61K 8/49, A61Q 5/08, A61Q 5/10, A61Q 11/00, C01B 15/08, C01B 15/12, C01B 15/037

(54) **VERWENDUNG VON HYDROXYPYRIDONEN ODER DEREN SALZEN ZUR STABILISIERUNG VON WASSERSTOFFPEROXID ODER WASSERSTOFFPEROXID FREISETZENDEN SUBSTANZEN**
USE OF HYDROXYPYRIDONES OR SALTS THEREOF FOR STABILIZING HYDROGEN PEROXIDE OR HYDROGEN PEROXIDE DONOR SUBSTANCES
UTILISATION D'HYDROXYPYRIDONES OU DE LEURS SELS POUR LA STABILISATION DE PEROXYDE D'HYDROGÈNE OU DE SUBSTANCES LIBÉRANT DU PEROXYDE D'HYDROGÈNE

(30) Priorität: 17.12.2010 DE 102010054865; 27.07.2010 DE 102010032371
(43) Veröffentlichungstag der Anmeldung: 05.06.2013
(73) Patentinhaber: Clariant Finance (BVI) Limited, Road Town, Tortola (VG)
(72) Erfinder: KLUG, Peter, 63762 Großostheim (DE); PILZ, Maurice, Frederic, 60329 Frankfurt am Main (DE); BACK, Ute, 63825 Blankenbach (DE)
(74) Vertreter: Paczkowski, Marcus
(86) Internationale Anmeldenummer: PCT/EP2011/003537
(87) Internationale Veröffentlichungsnummer: WO 2012/019689

(56) Entgegenhaltungen:
- EP-A1- 1 347 736
- FR-A1- 2 804 863

## Beschreibung

Die vorliegende Erfindung betrifft die Verwendung von Hydroxypyridonen oder deren Salzen zur Stabilisierung von Wasserstoffperoxid oder Wasserstoffperoxid freisetzenden Substanzen.

Zusammensetzungen und insbesondere wässrige Zusammensetzungen enthaltend Wasserstoffperoxid werden in verschiedenen Anwendungen benutzt. Sie werden in kosmetischen Mitteln z. B. als Bleichzusammensetzung für Haar, als Entwicklerkomponente in Haarfärbemitteln, aber auch als Komponente zur Haarfixierung in Dauerwellformulierungen verwendet. Weitere Anwendungen sind z. B. Zahnbleichzusammensetzungen. Auch in der industriellen Reinigung und in der Haushaltsreinigung und in der Textilbleiche sind Wasserstoffperoxid-haltige Zusammensetzungen vertreten.

Allerdings ist die Stabilität des Wasserstoffperoxids oder der Wasserstoffperoxid freisetzenden Substanzen in den Zusammensetzungen oftmals unbefriedigend.

Es bestand deshalb die Aufgabe, neue Stabilisatoren für Wasserstoffperoxid und Wasserstoffperoxid freisetzende Substanzen zur Verfügung zu stellen.

Überraschend wurde nun gefunden, dass diese Aufgabe durch die Verwendung von Hydroxypyridonen oder deren Salzen gelöst wird.

Gegenstand der Erfindung ist somit die Verwendung einer oder mehrerer Substanzen ausgewählt aus der Gruppe bestehend aus Hydroxypyridonen und deren Salzen (Komponente d)) zur Stabilisierung einer oder mehrerer Substanzen ausgewählt aus der Gruppe bestehend aus Wasserstoffperoxid und Wasserstoffperoxid freisetzenden Substanzen (Komponente a)).

Im Folgenden werden die eine oder die mehreren Substanzen ausgewählt aus der Gruppe bestehend aus Hydroxypyridonen und deren Salzen auch als Substanzen der Komponente d) bezeichnet.

Die eine oder mehreren Substanzen ausgewählt aus der Gruppe bestehend aus Wasserstoffperoxid und Wasserstoffperoxid freisetzenden Substanzen werden im Folgenden auch als Substanzen der Komponente a) bezeichnet.

Durch die erfindungsgemäße Verwendung werden das Wasserstoffperoxid und/oder die Wasserstoffperoxid freisetzenden Substanzen stabilisiert wodurch beispielsweise die Lagerstabilität entsprechender Zusammensetzungen erhöht wird. Durch die erhöhte Stabilität des Wasserstoffperoxids und/oder der Wasserstoffperoxid freisetzenden Substanzen in diesen Zusammensetzungen kann entweder die Wirkung der Inhaltsstoffe gesteigert werden wie z. B. deren Reinigungs- oder Bleichleistung, oder die Nutzungsdauer solcher Formulierungen gesteigert werden.

In EP 1 347 736 werden oxidative Zusammensetzungen zur Haarbehandlung beschrieben, die Stabilisatoren für Wasserstoffperoxid auf Basis Pyrophosphat, Stannaten, Phenacetin oder Oxychinolin oder deren Kombinationen enthalten.

Vorzugsweise ist die eine oder sind die mehreren Substanzen der Komponente a) ausgewählt aus der Gruppe bestehend aus Wasserstoffperoxid, Harnstoffperoxid, Perboraten, Persulfaten und Mischungen davon. Besonders bevorzugt ist die Substanz der Komponente a) Wasserstoffperoxid.

Vorzugsweise ist die eine oder sind die mehreren Substanzen der Komponente d) ausgewählt aus Verbindungen der Formel (I) und deren Salzen 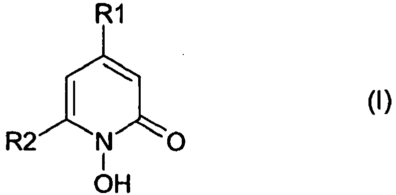 worin R1 H oder ein C₁-C₄ Alkylrest und R2 H, ein unsubstituierter oder mit Halogen substituierter, verzweigter oder unverzweigter C₁-C₂₀-Alkylrest, ein unsubstituierter oder mit Halogen substituierter C₅-C₈ Cycloalkylrest, ein unsubstituierter oder mit Halogen substituierter C₆-C₁₀ Arylrest oder ein unsubstituierter oder mit Halogen substituierter, verzweigter oder unverzweigter C₇-C₂₀ Aralkylrest ist.

Vorzugsweise sind die Reste R2 nicht mit Halogen substituiert.

In einer bevorzugten Ausführungsform der Erfindung liegt die eine oder liegen die mehreren Verbindungen der Komponente d) in Form der Säure (Verbindungen der Formel (I)) oder in Form ihrer Alkali-, Erdalkali- oder Aminsalze oder ihrer Salze mit polymeren Gegenionen vor.

In der einen oder in den mehreren Verbindungen der Formel (I) oder in ihren Salzen ist R1 vorzugsweise Methyl und R2 vorzugsweise Cyclohexyl oder 2,4,4-Trimethylpentyl.

Besonders bevorzugt liegen die Verbindungen der Formel (I) in Form ihrer Alkanolaminsalze und insbesondere bevorzugt in Form ihrer Monoethanolaminsalze vor. Beispiele für derartige Salze sind in DE 2234009 erwähnt.

Besonders bevorzugt sind dabei 4-Methyl-6-(2,4,4-trimethylpentyl)-1-hydroxy-2-pyridon, das Monoethanolaminsalz von 4-Methyl-6-(2,4,4-trimethylpentyl)-1-hydroxy-2-pyridon (Octopirox^{®}, Clariant) sowie 4-Methyl-6-(cyclohexyl)-1-hydroxy-2-pyridon und das Monoethanolaminsalz von 4-Methyl-6-(cyclohexyl)-1-hydroxy-2-pyridon (Ciclopirox^{®}, Sanofi-Aventis).

Diese Substanzen können anhand literaturbekannter Verfahren erhalten werden, vergleiche hierzu die in DE 2234009 genannten Referenzen.

Vorzugsweise findet die erfindungsgemäße Verwendung in wässrigen Zusammensetzungen statt.

Vorzugsweise ist das Wasser in einer Menge von 40 Gew.-% oder mehr und besonders bevorzugt in einer Menge von 50 Gew.-% oder mehr in den wässrigen Zusammensetzungen enthalten, bezogen auf das Gesamtgewicht der wässrigen Zusammensetzungen.

Die eine oder die mehreren Substanzen ausgewählt aus der Gruppe bestehend aus Wasserstoffperoxid und Wasserstoffperoxid freisetzenden Substanzen der Komponente a) sind vorzugsweise in Mengen von 0,5 bis 20 Gew.-%, besonders bevorzugt in Mengen von 1 bis 10 Gew.-%, insbesondere bevorzugt in Mengen von 1,5 bis 7 Gew.-% und außerordentlich bevorzugt in Mengen von 2 bis 7 Gew.-% in den wässrigen Zusammensetzungen enthalten, bezogen auf das Gesamtgewicht der wässrigen Zusammensetzungen. Hierunter wiederum bevorzugt ist die Substanz der Komponente a) Wasserstoffperoxid, welches vorzugsweise in Mengen von 0,5 bis 20 Gew.-%, besonders bevorzugt in Mengen von 1 bis 10 Gew.-%, insbesondere bevorzugt in Mengen von 1,5 bis 7 Gew.-% und außerordentlich bevorzugt in Mengen von 2 bis 7 Gew.-% in den wässrigen Zusammensetzungen enthalten ist, bezogen auf das Gesamtgewicht der wässrigen Zusammensetzungen.

In den wässrigen Zusammensetzungen ist die eine oder sind die mehreren Substanzen der Komponente d) in Mengen von vorzugsweise 0,1 bis 20 000 ppm (0,00001 bis 2 Gew.-%), besonders bevorzugt in Mengen von 0,5 bis 1 000 ppm (0,00005 bis 0,1 Gew.-%) und insbesondere bevorzugt in Mengen von 0,5 bis 100 ppm (0,00005 bis 0,01 Gew.-%) enthalten, bezogen auf das Gesamtgewicht der wässrigen Zusammensetzungen.

Die Hydroxypyridone oder ihre Salze können bei der erfindungsgemäßen Verwendung mit weiteren Stabilisatoren kombiniert werden. Weitere geeignete Stabilisatoren sind z. B. Polyphosphate oder deren Alkali- oder Erdalkalimetallsalze, Alkali- oder Erdalkalistannate, Phenacetin und seine Säuresalze sowie Oxychinolin und seine Säuresalze.

Die wässrigen Zusammensetzungen können als weitere Hilfs- und Zusatzstoffe Ölkörper, Silikonöle, Wachse, Tenside, Emulgatoren, Co-Emulgatoren, Solubilisatoren, kationische Polymere, Filmbildner, Überfettungsmittel, Rückfetter, antimikrobielle Wirkstoffe, Feuchthaltemittel, Lösemittel, Farbstoffe, Duftstoffe, Perlglanzmittel und/oder Trübungsmittel enthalten.

Die wässrigen Zusammensetzungen können organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin, 1,2- und 1,3-Propandiol und Mischungen aus den genannten Alkoholen eingesetzt. Weitere bevorzugte Alkohole sind Polyethylenglykole mit einer relativen Molekülmasse unter 2 000. Insbesondere ist ein Einsatz von Polyethylenglykol mit einer relativen Molekülmasse zwischen 200 und 600 und in Mengen bis zu 45,0 Gew.-% und von Polyethylenglykol mit einer relativen Molekülmasse zwischen 400 und 600 in Mengen von 5,0 bis 25,0 Gew.-% bevorzugt. Weitere geeignete Lösungsmittel sind beispielsweise Triacetin (Glycerintriacetat) und 1-Methoxy-2-propanol.

Bei den wässrigen Zusammensetzungen kann es sich z. B. auch um wässrigtensidische oder wässrig-alkoholische Zusammensetzungen oder um Emulsionen handeln.

Als Säuren oder Laugen zur pH-Wert Einstellung der wässrigen Zusammensetzungen werden vorzugsweise Mineralsäuren, insbesondere HCl, anorganische Basen, insbesondere NaOH oder KOH, und organische Säuren, insbesondere Zitronensäure, verwendet.

Vorzugsweise weisen die wässrigen Zusammensetzungen einen pH-Wert von 2 bis 11, besonders bevorzugt von 7 bis 11, insbesondere bevorzugt von 8 bis 11 und außerordentlich bevorzugt von 8,5 bis 11, auf.

Die erfindungsgemäßen Stabilisierung des Wasserstoffperoxids oder der Wässerstoffperoxid-freisetzenden Substanzen kann z. B. bei folgenden Anwendungen ausgenutzt werden: in Bleichzusammensetzungen für das Haar oder die Zähne, in oxidativen Haarfarben, bei der Verwendung des Wasserstoffperoxids oder der Wasserstoffperoxid-freisetzenden Substanzen als Fixierkomponente für Dauerwellformulierungen, in Haushaltsreinigern, in oxidativen Reinigerformulierungen, in Zusammensetzungen zum oxidativen Bleichen von Fasern oder Textilien, in Vorwaschsprays, Fleckentfemern, Oberflächenreinigern oder Toilettenreinigern.

Die nachfolgenden Beispiele und Anwendungen sollen die Erfindung näher erläutern, ohne sie jedoch darauf zu beschränken. Bei allen Prozentangaben handelt es sich um Gewichts-% (Gew.-%), sofern nicht explizit anders angegeben.

### Versuchsbeispiele:

### Beispiel 1:

Wasserstoffperoxidlösung von Solvay (35 Gew.-% in Wasser) bzw. von Merck (35 Gew.-% in Wasser) wurde auf einen Wasserstoffperoxidgehalt von etwa 6,0 Gew.-% mit vollentsalztem Wasser verdünnt und mit Natronlauge (20 Gew.-%) auf einen pH-Wert von 9,0 eingestellt. Weitere Lösungen wurden jeweils mit 8 ppm 4-Methyl-6-(2,4,4-trimethylpentyl)-1-hydroxy-2-pyridon (Additiv A, gelöst in Propylenglykol) additivert. Die Lösungen wurden bei Raumtemperatur (20 °C) und 40 °C 1 Woche gelagert und der Wasserstoffperoxidgehalt vor und nach Lagerung gemessen (siehe Tabelle 1).

**Tabelle 1 Ergebnisse der Messung des Wasserstoffperoxidgehalts**

| Wasserstoffperoxid | Additiv A | Wasserstoffperoxidgehalt sofort | Wasserstoffperoxidgehalt nach 1 Woche 20 °C | Wasserstoffperoxidgehalt nach 1 Woche 40 °C |
|---|---|---|---|---|
| | | [Gew.-%] | [Gew.-%] | [Gew.-%] |
| Solvay (35 Gew.-%) | Nein | 6,3 | 4,8 | < 0,1 |
| Solvay (35 Gew.-%) | Ja | 6,0 | 6,1 | 5,9 |
| Merck (35 Gew.-%) | Nein | 5,9 | 3,9 | 2,0 |
| Merck (35 Gew.-%) | ja | 6,1 | 6,1 | 5,8 |

### Beispiel 2:

Wasserstoffperoxidlösung von Solvay (35 Gew.-% in Wasser) bzw. von Merck (35 Gew.-% in Wasser), eine Lösung von Sodium C₁₄₋₁₇ Alkyl sec. Sulfonate (Hostapur^{®} SAS 30) und vollentsalztes Wasser wurden so gemischt, dass ein Wasserstoffperoxidgehalt von etwa 6,0 Gew.-% sowie ein Gehalt an Sodium C₁₄₋₁₇ Alkyl sec. Sulfonate von 5,0 Gew.-% resultierte. Danach wurde mit Natronlauge (20 Gew.-%) auf einen pH-Wert von 9,0 eingestellt. Weitere Lösungen wurden jeweils mit 7 ppm 4-Methyl-6-(2,4,4-trimethylpentyl)-1-hydroxy-2-pyridon (Additiv A, gelöst in Propylenglykol) additivert. Die Lösungen wurden bei Raumtemperatur und 40 °C 1 Woche gelagert und der Wasserstoffperoxidgehalt vor und nach Lagerung gemessen (siehe Tabelle 2).

**Tabelle 2 Ergebnisse der Messung des Wasserstoffperoxidgehalts**

| Wasserstoffperoxid | Additiv A | Wasserstoffperoxidgehalt sofort | Wasserstoffperoxidgehalt nach 1 Woche 20 °C | Wasserstoffperoxidgehalt nach 1 Woche 40 °C |
|---|---|---|---|---|
| | | [Gew.-%] | [Gew.-%] | [Gew.-%] |
| Solvay (35 Gew.-%) | Nein | 6,1 | 3,7 | 0,1 |
| Solvay (35 Gew.-%) | Ja | 6,0 | 6,1 | 5,8 |
| Merck (35 Gew.-%) | Nein | 5,9 | 4,2 | 1,4 |
| Merck (35 Gew.-%) | ja | 6,0 | 6,1 | 5,2 |

Die Ergebnisse der Beispiele 1 und 2 zeigen, dass 4-Methyl-6-(2,4,4 trimethylpentyl)-1-hydroxy-2-pyridon sowohl bei Raumtemperatur als auch bei 40 °C die Lagerstabilität von Wasserstoffperoxidlösungen bei hohem pH-Wert signifikant steigern kann.

### Beispiel 3: Wasserstoffperoxid-Gel verdickt mit einem Sulfonat-Polymer

### Rezeptur:

| | |
|---|---|
| Wasser, vollentsalzt | ad 100 Gew.-% |
| Wasserstoffperoxidlösung (Solvay, 35 Gew.-%ig, wässrig) | 17 Gew.-% |
| Aristoflex^{®} AVS | 1,0 Gew-% |
| Sodium Acryloyldimethyltaurate/VP Crosspolymer 4-Methyl-6-(2,4,4-trimethylpentyl)-1-hydroxy-2-pyridon | 0,00081 Gew.-% |

Die Formulierung wurde durch Lösen des polymeren Verdickers in Wasser und anschließendes Einmischen der Wasserstoffperoxidlösung hergestellt. Der jeweilige Start-pH-Wert wurde anschließend mit 10 Gew.-%iger wässriger Natronlauge eingestellt. Die Formulierung wurde jeweils mit und ohne Zugabe von 8,1 ppm (0,00081 Gew.-%) 4-Methyl-6-(2,4,4-trimethylpentyl)-1-hydroxy-2-pyridon hergestellt.

Hierzu wurden 10 ml einer 0,1 Gew.-%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Gleichzeitig wurde jeweils der Wasserstoffperoxid-Gehalt iodometrisch bestimmt (siehe Tabelle 3).

**Tabelle 3 Ergebnisse der Messung des Wasserstoffperoxid-Gehalts**

| Start-pH | 8,1 ppm (= 0,00081 Gew.-%) 4-Methyl-6-(2,4,4-trimethylpentyl)-1-hydroxy-2-pyridon | Wasserstoffperoxid-Gehalt sofort [Gew.-%] | Wasserstoffperoxid-Gehalt nach 2 Wochen 25 °C [Gew.-%] |
|---|---|---|---|
| 9,0 | nein | 6.0 | 0,4 |
| 9,0 | ja | 6,0 | 6,0 |

Aus der Tabelle 3 erkennt man, dass der Wasserstoffperoxidgehalt der Lösung ohne Stabilisator vom Startwert 6,0 Gew.-% auf 0,4 Gew.-% abfällt, während mit Stabilisator noch der Anfangswert von 6,0 Gew.-% erhalten blieb.

Ähnliche Ergebnisse wie in den Beispielen 1-3 beschrieben wurden auch bei Einsatz von 10 ppm 4-Methyl-6-(2,4,4-trimethylpentyl)-1-hydroxy-2-pyridon, Monoethanolaminsalz (Octopirox^{®}) erhalten.

## Patentansprüche

1. Verwendung einer oder mehrerer Substanzen ausgewählt aus der Gruppe bestehend aus Hydroxypyridonen und deren Salzen (Komponente d)) zur Stabilisierung einer oder mehrerer Substanzen ausgewählt aus der Gruppe bestehend aus Wasserstoffperoxid und Wasserstoffperoxid freisetzenden Substanzen (Komponente a)).

2. Verwendung nach Anspruch 1, **dadurch gekennzeichnet, dass** die eine oder die mehreren Substanzen der Komponente a) ausgewählt sind aus der Gruppe bestehend aus Wasserstoffperoxid, Harnstoffperoxid, Perboraten, Persulfaten und Mischungen davon.

3. Verwendung nach Anspruch 1 oder 2, **dadurch gekennzeichnet, dass** die Substanz der Komponente a) Wasserstoffperoxid ist.

4. Verwendung nach einem oder mehreren der Ansprüche 1 bis 3, **dadurch gekennzeichnet, dass** die eine oder die mehreren Substanzen der Komponente d) ausgewählt sind aus Verbindungen der Formel (I) und deren Salzen 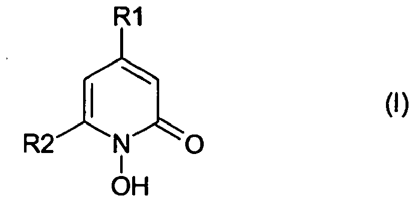 worin R1 H oder ein C₁-C₄ Alkylrest und R2 H, ein unsubstituierter oder mit Halogen substituierter, verzweigter oder unverzweigter C₁-C₂₀-Alkylrest, ein unsubstituierter oder mit Halogen substituierter C₅-C₈ Cycloalkylrest, ein unsubstituierter oder mit Halogen substituierter C₆-C₁₀ Arylrest oder ein unsubstituierter oder mit Halogen substituierter, verzweigter oder unverzweigter C₇-C₂₀ Aralkylrest ist.

5. Verwendung nach einem oder mehreren der Ansprüche 1 bis 4, **dadurch gekennzeichnet, dass** die eine oder die mehreren Verbindungen der Komponente d) in Form der Säure oder in Form ihrer Alkali-, Erdalkali- oder Aminsalze oder in Form ihrer Salze mit polymeren Gegenionen vorliegen.

6. Verwendung nach Anspruch 4 oder 5, **dadurch gekennzeichnet, dass** in der einen oder in den mehreren Verbindungen der Formel (I) oder in ihren Salzen R1 Methyl ist und R2 Cyclohexyl oder 2,4,4-Trimethylpentyl ist.

7. Verwendung nach einem oder mehreren der Ansprüche 1 bis 6, **dadurch gekennzeichnet, dass** die Stabilisierung in wässrigen Zusammensetzungen stattfindet.

8. Verwendung nach Anspruch 7, **dadurch gekennzeichnet, dass** die Substanz der Komponente a) Wasserstoffperoxid ist und das Wasserstoffperoxid in Mengen von 0,5 bis 20 Gew.-% in der wässrigen Zusammensetzung enthalten ist, bezogen auf das Gesamtgewicht der wässrigen Zusammensetzung.

9. Verwendung nach Anspruch 7 oder 8, **dadurch gekennzeichnet, dass** die eine oder die mehreren Substanzen der Komponente d) in Mengen von 0,1 ppm bis 2 Gew.-% in der wässrigen Zusammensetzung enthalten sind, bezogen auf das Gesamtgewicht der wässrigen Zusammensetzung.

10. Verwendung nach 9, **dadurch gekennzeichnet, dass** die eine oder die mehreren Substanzen der Komponente d) in Mengen von 0,5 bis 100 ppm in der wässrigen Zusammensetzung enthalten sind, bezogen auf das Gesamtgewicht der wässrigen Zusammensetzung.

11. Verwendung nach einem oder mehreren der Ansprüche 7 bis 10, **dadurch gekennzeichnet, dass** die wässrige Zusammensetzung einen pH-Wert von 2 bis 11 aufweist.

12. Verwendung nach Anspruch 11, **dadurch gekennzeichnet, dass** die wässrige Zusammensetzung einen pH-Wert von 7 bis 11 aufweist.

## Claims

1. The use of one or more substances selected from the group consisting of hydroxypyridones and salts thereof (component d)) for stabilizing one or more substances selected from the group consisting of hydrogen peroxide and hydrogen peroxide donor substances (component a)).

2. The use as claimed in claim 1, wherein the one or more substances of component a) are selected from the group consisting of hydrogen peroxide, urea peroxide, perborates, persulfates and mixtures thereof.

3. The use as claimed in claim 1 or 2, wherein the substance of component a) is hydrogen peroxide.

4. The use as claimed in one or more of claims 1 to 3, wherein the one or more substances of component d) are selected from compounds of the formula (I) and salts thereof 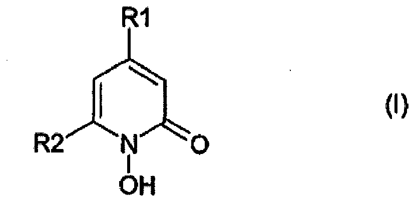 in which R1 is H or a C₁-C₄-alkyl radical and R2 is H, an unsubstituted or halogen-substituted, branched or unbranched C₁-C₂₀-alkyl radical, an unsubstituted or halogen-substituted C₅-C₈-cycloalkyl radical, an unsubstituted or halogen-substituted C₆-C₁₀-aryl radical or an unsubstituted or halogen-substituted, branched or unbranched C₇-C₂₀-aralkyl radical.

5. The use as claimed in one or more of claims 1 to 4, wherein the one or more compounds of component d) are present in the form of the acid or in the form of their alkali metal, alkaline earth metal or amine salts or in the form of their salts with polymeric counterions.

6. The use as claimed in claim 4 or 5, wherein, in the one or more compounds of the formula (I) or in their salts, R1 is methyl and R2 is cyclohexyl or 2,4,4-trimethylpentyl.

7. The use as claimed in one or more of claims 1 to 6, wherein the stabilization takes place in aqueous compositions.

8. The use as claimed in claim 7, wherein the substance of component a) is hydrogen peroxide and the hydrogen peroxide is present in amounts of from 0.5 to 20% by weight in the aqueous composition, based on the total weight of the aqueous composition.

9. The use as claimed in claim 7 or 8, wherein the one or more substances of component d) are present in amounts of from 0.1 ppm to 2% by weight in the aqueous composition, based on the total weight of the aqueous composition.

10. The use as claimed in claim 9, wherein the one or more substances of component d) are present in amounts of from 0.5 to 100 ppm in the aqueous composition, based on the total weight of the aqueous composition.

11. The use as claimed in one or more of claims 7 to 10, wherein the aqueous composition has a pH of from 2 to 11.

12. The use as claimed in claim 11, wherein the aqueous composition has a pH of from 7 to 11.

## Revendications

1. Utilisation d'une ou de plusieurs substances choisies dans le groupe constitué par les hydroxypyridones et leurs sels (composant d)) pour la stabilisation d'une ou de plusieurs substances choisies dans le groupe constitué par le peroxyde d'hydrogène et les substances libérant du peroxyde d'hydrogène (composant a)).

2. Utilisation selon la revendication 1, **caractérisée en ce que** la ou les substances du composant a) sont choisies dans le groupe constitué par le peroxyde d'hydrogène, le peroxyde d'urée, les perborates, les persulfates et leurs mélanges.

3. Utilisation selon la revendication 1 ou 2, **caractérisée en ce que** la substance du composant a) est le peroxyde d'hydrogène.

4. Utilisation selon une ou plusieurs des revendications 1 à 3, **caractérisée en ce que** la ou les substances du composant d) sont choisies parmi les composés de formule (I) et leurs sels 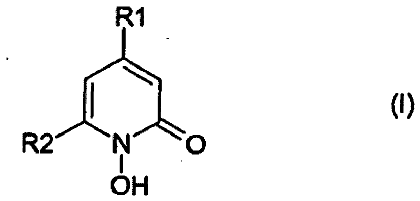 dans laquelle R1 représente H ou un radical alkyle en C₁-C₄ et R2 représente H, un radical alkyle en C₁-C₂₀ non substitué ou substitué avec halogène, ramifié ou non ramifié, un radical cycloalkyle en C₅-C₈ non substitué ou substitué avec halogène, un radical aryle en C₆-C₁₀ non substitué ou substitué avec halogène, ou un radical aralkyle en C₇-C₂₀ non substitué ou substitué avec halogène, ramifié ou non ramifié.

5. Utilisation selon une ou plusieurs des revendications 1 à 4, **caractérisée en ce que** le ou les composés du composant d) se présentent sous la forme de l'acide ou sous la forme de leurs sels alcalins, alcalino-terreux ou d'amines, ou sous la forme de leurs sels avec des contre-ions polymères.

6. Utilisation selon la revendication 4 ou 5, **caractérisée en ce que** R1 représente méthyle et R2 représente cyclohexyle ou 2,4,4-triméthylpentyle dans le ou les composés de formule (I) ou dans leurs sels.

7. Utilisation selon une ou plusieurs des revendications 1 à 6, **caractérisée en ce que** la stabilisation a lieu dans des compositions aqueuses.

8. Utilisation selon la revendication 7, **caractérisée en ce que** la substance du composant a) est le peroxyde d'hydrogène et le peroxyde d'hydrogène est contenu en quantités allant de 0,5 à 20 % en poids dans la composition aqueuse, par rapport au poids total de la composition aqueuse.

9. Utilisation selon la revendication 7 ou 8, **caractérisée en ce que** la ou les substances du composant d) sont contenues en quantités allant de 0,1 ppm à 2 % en poids dans la composition aqueuse, par rapport au poids total de la composition aqueuse.

10. Utilisation selon la revendication 9, **caractérisée en ce que** la ou les substances du composant d) sont contenues en quantités allant de 0,5 à 100 ppm dans la composition aqueuse, par rapport au poids total de la composition aqueuse.

11. Utilisation selon une ou plusieurs des revendications 7 à 10, **caractérisée en ce que** la composition aqueuse présente un pH allant de 2 à 11.

12. Utilisation selon la revendication 11, **caractérisée en ce que** la composition aqueuse présente un pH allant de 7 à 11.
